# EUROPÄISCHE PATENTSCHRIFT

(11) **EP 0 770 687 B1**
(45) Veröffentlichungstag und Bekanntmachung des Hinweises auf die Patenterteilung: **23.01.2002**
(21) Anmeldenummer: 96117280.6
(22) Anmeldetag: 28.10.1996
(51) Int. Cl.: C12P 17/12

(54) **Verfahren zur Herstellung von Nikotinsäureamid**
Process for the preparation of nicotinic acid amide
Procédé pour la préparation de l'amide de l'acide nicotinique

(30) Priorität: 01.11.1995 CH 309095
(43) Veröffentlichungstag der Anmeldung: 02.05.1997
(73) Patentinhaber: Lonza AG, 3930 Visp (CH)
(72) Erfinder: Heveling, Josef, Dr., 3904 Naters (Kanton Wallis) (CH); Armbruster, Erich, Dr., 3904 Naters (Kanton Wallis) (CH); Utiger, Lukas, Dr., 3912 Termen (Kanton Wallis) (CH); Rohner, Markus, Dr., CZ 28161 Kourim (CZ); Dettwiler, Hans-Rudolf, Dr., 3902 Brig-Glis (Kanton Wallis) (CH); Chuck, Roderick John, Dr., 3902 Brig-Glis (Kanton Wallis) (CH)
(74) Vertreter: Weinhold, Peter, Dr.rer.nat. Dipl.-Chem.

(56) Entgegenhaltungen:
- EP-A- 0 307 926
- WO-A-94/22824
- WO-A-95/32055
- US-A- 5 028 713
- CALUWADEWA DEEPAL M. ET AL.: "Nitrilase-Catalyzed Production of Nicotinic Acid from 3-Cyanopyridine in Rhodococcus rhodochrous J1." APPL. ENVIRON. MICROBIOL., Bd. 54, Nr. 4, 1988, Seiten 1030-1032, XP002069032
- NEUMüLLER, OTTO-ALBRECHT: "Römpps Chemie-Lexikon, S. 4690,4691,4731", 1988, FRANCKH'SCHE VERLAGSHANDLUNG , STUTTGART

## Beschreibung

Die Erfindung betrifft ein neues Verfahren zur Herstellung von Nikotinsäureamid.

Zur Herstellung von Nikotinsäureamid, einem für Mensch und Tier essentiellen Vitamin des B-Komplexes, sind zahlreiche Verfahren bekannt. Grosstechnische Bedeutung erlangt haben im wesentlichen lediglich zwei Verfahren, nämlich die Salpetersäure-Oxidation von Alkylpyridinen oder die Ammonoxidation von Alkylpyridinen (vgl. Ullmann's Encyklopädie der technischen Chemie, 4. Auflage, Bd. 23, S. 708 ff. bzw. Bd. 19, S. 602 ff.). Obwohl die Salpetersäure-Oxidation, speziell von 2-Methyl-5-ethylpyridin, ein sehr selektives Verfahren darstellt, beinhaltet sie ein Risikopotential, für dessen Minimierung hochqualifiziertes Personal, optimale Infrastruktur und ein hoher know how-Standard unabdingbare Voraussetzung sind. Für einen Technologietransfer, z.B. an Orte, wo diese genannten Voraussetzungen nur teilweise realisierbar sind, ist das genannte Verfahren folglich ungeeignet.
Die Ammonoxidation, speziell von 3-Picolin, hat grosstechnisch bisher nicht annähernd die Bedeutung der Salpetersäure-Oxidation erhalten, obwohl zahlreiche Publikationen quantitative Umsätze bei Ausbeuten von über 90% beschreiben (vgl. Ullmann's Encyklopädie der technischen Chemie, 4. Auflage, Bd. 19, S. 602 ff.). Die US-A-5 028 713 beschreibt die Ammonoxidation von Methylpyridinen, wobei als Katalysator ein Komplex mit den Oxiden von P, V und Mo eingesetzt wird, der gegebenenfalls weitere katalytische wirksame Metalle enthalten kann. Wesentliche Voraussetzung für einen industriell brauchbaren Katalysator sind aber nicht nur seine Umsetzungsrate und seine Selektivität, sondern ebenso seine Belastbarkeit (Menge Ausgangsprodukt / Katalysatorvolumen / Zeit = kg l⁻¹h⁻¹) und seine Standzeit. Vor allem bezüglich der letzten beiden Kriterien vermögen die bekannten Ammonoxidationskatalysatoren aus dem Stand der Technik nicht zu genügen.

Ein biologisches Verfahren zur Herstellung von Nikotinsäureamid wird in der EP-A-0 307 926 und bei Caluwadewa Deepal, M. et al. (Appl. Environ. Microbiol., 54, 1030-1032, 1988) beschrieben. Bei diesem Verfahren wird 3-Cyanpyridin mittels einer aus Mikroorganismen der Spezies *Rhodococcus rhodochrous* stammenden Nitrilhydratase in das Nikotinsäureamid überführt.

Die Aufgabe der vorliegenden Erfindung bestand darin, ein industriell anwendbares Verfahren zu entwickeln, das einerseits auf einer relativ einfach beherrschbaren Technologie beruht und andererseits alle Kriterien und Anforderungen, die an ein wirtschaftliches Verfahren zu stellen sind, erfüllt.

Diese Aufgabe wurde mit den Verfahren gemäß den Ansprüchen 1 und 10 gelöst.

Erfindungsgemäss wird in der ersten Stufe
a) 2-Methyl-1,5-diaminopentan in der Gasphase bei 300 ° - 400 °C und bei 0 - 10 bar Überdruck durch Überleiten über einen Katalysator, der als aktive Komponente mindestens ein Oxid von Al und/oder Si enthält, an der Oberfläche ein Verhältnis von sauren Zentren zu basischen Zentren von über 2 aufweist und dessen spezifische Oberfläche über 40m²/g liegt, in 3-Methylpiperidin überführt und unmittelbar anschliessend bei 220 ° - 400 °C über einen Dehydrierungskatalysator geleitet und in 3-Picolin überführt,
   darauf in der zweiten Stufe
b) 3-Picolin in Gegenwart von Ammoniak und Sauerstoff enthaltendem Gas bei 280 ° - 400 °C über einen Ammonoxidationskatalysator geleitet, der aus den Oxiden von Vanadium, Titan, Zirkon und Molybdän in einem Molverhältnis V₂O₅ zu TiO₂ zu ZrO₂ von 1:1:2 bis 1:12:25 und einem MoO₃-Gehalt, bezogen auf V₂O₅, von 0,54 Gew.% bis 2,6 Gew.%, besteht, und resultierendes 3-Cyanpyridin
   schliesslich in der dritten Stufe
c) mittels Mikroorganismen der Gattung *Rhodococcus* in das Endprodukt überführt.

Die erste Verfahrensstufe, die Herstellung von 3-Picolin aus 2-Methyl-1,5-diaminopentan, ist in der WO 94/22824 ausführlich beschrieben.

Zweckmäßig wird als Dehydrierungskatalysator ein Edelmetallkatalysator auf einem Träger verwendet.

Das anfallende 3-Picolin kann ohne Zwischenreinigung direkt in die Ammonoxidationsstufe eingespiesen werden. Vorzugsweise erfolgt jedoch eine beispielsweise destillative Zwischenreinigung, welche die Katalysatorstandzeit in der Folgestufe positiv beeinflusst.

Die Ammonoxidation in der zweiten Stufe ist Gegenstand der PCT-Anmeldung PCT/EP 95/01945 (WO 95/32055).

Als bevorzugter Ammonoxidationskatalysator wird eine Katalysatorzusammensetzung verwendet, die aus den Oxiden von Vanadium, Titan, Zirkon und Molybdän in einem Molverhältnis V₂O₅ zu TiO₂ zu ZrO₂ von 1:3:4 bis 1:8:16 und einem MoO₃-Gehalt, bezogen auf V₂O₅, von 0,54 Gew.% bis 1,20 Gew.% besteht.

Die Bereitstellung des Katalysators ist ausführlich in der obengenannten WO 95/32055 beschrieben.

Als Sauerstoff enthaltendes Gas wird bevorzugt Luft verwendet, nachdem Luft den Vorteil aufweist, dass der Sauerstoff bereits mit Inertgasen verdünnt vorliegt. Allerdings kann der Partialdruck von Sauerstoff weiter reguliert werden durch Zumischung von Inertgas wie Stickstoff oder von durch Recyclierung gewonnenen Sauerstoff-freien Prozessgasen.

Zweckmässig werden die Reaktanden 3-Picolin, Ammoniak und Sauerstoff enthaltendes Gas (berechnet auf O₂) gasförmig in einem Molverhältnis von 1.1:1,5 bis 1:8,5:60 bei 280°-400 °C, vorzugsweise bei 310 ° - 380 °C über den Katalysator geleitet. Bevorzugte molare Zusammensetzung des Zufuhrgases ist 3-Picolin, Ammoniak und Sauerstoff enthaltendes Gas (berechnet auf O₂) 1:1:1,5 bis 1:4:25.
Weiterhin bevorzugt werden 3-Picolin, Ammoniak und Sauerstoff enthaltendes Gas (berechnet auf O₂) in einem Molverhältnis von 1:1:10 bis 1:4:60 über den Katalysator geleitet.

Wasser kann die Aktivität des Katalysators günstig beeinflussen und wird zweckmässig mit einem Molverhältnis auf 3-Picolin zwischen 0 und 5, vorzugsweise bei ca. 1,5, über den Katalysator geleitet.

In dieser zweiten Stufe werden bei einer Katalysatorbelastung von 50 bis zu 150g l⁻¹h⁻¹ 3-Picolin Ausbeuten an 3-Cyanpyridin von bis zu 99% erreicht. Die Katalysatorstandzeit ist mit mindestens einem Jahr ebenfalls ausserordentlich hoch.
Im Vergleich zum Stand der Technik konnte mit dem vorliegenden Ammonoxidationsverfahren, als Bestandteil des erfindungsgemässen Verfahrens, ein Verfahren entwickelt werden, das allen Kriterien einer industriellen Umsetzung genügt.

Das resultierende 3-Cyanpyridin kann in Form einer wässrigen Lösung direkt oder nach einem Aufarbeitungsschritt, z. B. einer Kristallisation, Extraktion oder Destillation,der Biohydrolyse zugeführt werden. Eine bevorzugte Aufarbeitung besteht in der Gegenstromextraktion des 3-Cyanpyridins mit z. B. Toluol und anschliessender Vakuumdestillation. Das verwendete Lösungsmittel, z. B. Toluol, kann vollständig recyclisiert werden.

Die Biohydrolyse von 3-Cyanpyridin als Substrat zum Nikotinsäureamid erfolgt zweckmässig mit Mikroorganismen der Spezies *Rhodococcus rhodochrous, Rhodococcus sp.* S - 6 oder *Rhodococcus equi*, vorzugsweise mit Mikroorganismen der Spezies *Rhodococcus sp*. S - 6 (FERM BP-687), *Rhodococcus rhodochrous* J1 (FERM BP-1478) oder mit Mikroorganismen der Spezies *Rhodococcus equi* TG328 (FERM BP-3791)*. Insbesondere wird die Umsetzung mittels Mikroorganismen der Spezies *Rhodococcus rhodochrous* (FERM BP- 1478) durchgeführt. Die Mikroorganismen der Spezies *Rhodococcus sp.* S - 6, *Rhodococcus rhodochrous* J1 und *Rhodococcus equi* TG328 sind in der Literatur beschriebene Mikroorganismen. *Rhodococcus rhodochrous* J1 (FERM BP-1478) ist in der EP-B 307 926, *Rhodococcus sp*. S - 6 (FERM BP-687) in der EP-A 0 188 316 und *Rhodococcus equi* TG328 (FERM BP-3791) in der US-PS 5 258 305 ausführlich beschrieben. Sämtliche genannten Mikroorganismen wurden hinterlegt beim National Institute of Bioscience and Human-Technology (NIBH), Agency of Industrial Science and Technology, Ministry of International Trade and Industry, 1-3, Higashi 1-chome, Tsukuba-shi, Ibaraki-ken 305; Japan.
*)(auch hinterlegt bei der Deutschen Sammlung von Mikroorganismen (DSM) und Zellkulturen GmbH, Mascheroder Weg 1B, 3300 Braunschweig, BRD am 13.9.1991 unter der Eingangsnummer DSM 6710)

Für das Verfahren ebenfalls geeignet sind die funktionell aequivalenten Varianten und Mutanten dieser Mikroorganismen. Unter "funktionell aequivalenten Varianten und Mutanten" werden Mikroorganismen verstanden, die im wesentlichen dieselben Eigenschaften und Funktionen wie die Ursprungsmikroorganismen besitzen. Derartige Varianten und Mutanten können zufällig z. B. durch UV-Bestrahlung gebildet werden.

Üblicherweise werden die Mikroorganismen vor der eigentlichen Biotransformation entsprechend der EP-B 307 926 kultiviert (angezüchtet) und die wirksamen Enzyme induziert. Vorzugsweise erfolgt die Biotransformation mit, auf fachmännisch übliche Weise, immobilisierten Mikroorganismen-Zellen.

Zweckmässig wird die Biotransformation in einem pH-Bereich von 6 bis 10, vorzugsweise in einem pH-Bereich von 6,5 bis 8,5, durchgeführt. Die pH-Einstellung erfolgt dabei zweckmässig über einen geeigneten Phosphatpuffer.

Die Biotransformation kann bei einer Temperatur von 5 bis 50 °C, vorzugsweise von 15 bis 30 °C, durchgeführt werden.

Bevorzugt erfolgt die Biohydrolyse des zweckmässig mit einem Gehalt von 5 bis 30 Gew.% in wässriger Lösung vorliegenden 3-Cyanpyridins in einer Reaktorkaskade, bestehend aus 2 bis 5 miteinander verbundenen, gerührten Reaktoren, welche jeweils den Biokatalysator enthalten. Besonders bevorzugt werden Kaskaden mit 3 bis 4 Rührwerken verwendet.
Der 3-Cyanpyridin-Gehalt in der wässrigen Lösung bewegt sich besonders vorteilhaft zwischen 10 und 20 Gew.%.

Nach einer Verweilzeit von 5 bis 30 Stunden kann aus dem Produktstrom das Nikotinsäureamid
z. B. durch Kristallisation gewonnen werden. Bevorzugt wird die Reaktionslösung über Aktivkohle oder einem Polystyrolharz (z. B. Amberlite) gereinigt und das Nikotinsäureamid auf fachmännische Weise aus der wässrigen Phase isoliert.

Die Umsetzung in der Biohydrolyse ist praktisch quantitativ und liefert ein Nikotinsäureamid mit einem Gehalt von über 99,5 %.

### Beispiele:

### Beispiel 1a

### MPDA (Methyldiaminopentan) zu 3-Picolin:

In einen Reaktor (13 mm ∅) wurden 4 g eines Pd-Katalysators (1% Pd/Al₂O₃) eingefüllt und darüber 3 g H-ZSM-5 (54,5% Pentasil (Si/Al = 18) + 45,5% Binder). (Das Edukt wurde immer von oben in den Reaktor eingespeist.) Die Reaktionsbedingungen waren: Temperatur: 305 - 320 °C, 15 ml/min N₂, Druck: 5 bar. Im Temperaturbereich 305 - 320 °C und bei einer MHSV (Katalysatorbelastung) von 0,6 g/(g·h) wurden Ausbeuten von bis zu 97% 3-Picolin erreicht, wobei als einziges weiteres Produkt 2,9% MPI (Methylpiperidin) gefunden wurden. Es erfolgte also ein vollständiger Umsatz des MPDA zu erwünschten Produkten. Innerhalb von 10 Tagen wurde keine Desaktivierung der Katalysatoren beobachtet. Statt N2 kann auch H2 als Trägergas verwendet werden.

### Beispiel 1b

### Herstellung von 3-Picolin mit zwei getrennten Reaktoren und kommerziellem MPDA (MPDA zu 3 Picolin in 2 Stufen mit Isolierung von MPI):

**1. Stufe:** In einen Reaktor (13 mm ∅) wurden 3 g ZSM-5 in der Ammonium-Form (Korngrösse: 0,5 - 1 mm) eingefüllt. MPDA wurde verdampft und mit einem Trägergasstrom von 15 ml/min N₂ bei einem Druck von 5 bar und einer Temperatur von 335 °C über den Katalysator geleitet. Die MHSV betrug 4,2 g MPDA pro Gramm Katalysator pro Stunde. Das verwendete MPDA war ein kommerzielles Produkt, das von der Firma Du Pont de Nemours unter dem Handelsnamen Dytek A erhältlich ist. Der Versuch lief über 280 Stunden. Eine Desaktivierung des Katalysators war nicht zu beobachten. Das Produkt wurde kondensiert, und das gebildete Ammoniak konnte entweichen. Die Ausbeuten an MPI waren praktisch quantitativ (>99,5%).
**2. Stufe:** In einen Reaktor (13 mm ∅) wurden 10 g eines Pd-MgCl₂/Al₂O₃-Dehydrierkatalysators eingefüllt. Das MPI aus der ersten Stufe wurde dampfförmig mit einem Trägergasstrom von 15 ml/min N2 bei einem Druck von 1 bar und einer Temperatur von 280 °C über den Katalysator geleitet. Die MHSV betrug 0,23 g MPI pro Gramm Katalysator pro Stunde. Der Versuch lief über 190 Stunden. Eine Desaktivierung des Katalysators war nicht zu beobachten. Nach 190 h wurde gaschromatographisch folgende Produktzusammensetzung festgestellt: 99,3% 3-Picolin, 0,4% MPI.

### Beispiel 1c

### Herstellung von 3-Picolin mit zwei getrennten Reaktoren und kommerziellem MPDA (MPDA zu 3-Picolin in 2 Stufen ohne Isolierung von MPI):

In einen Reaktor (13 mm ∅) wurden 3 g NH₄-ZSM-5 (Korngrösse: 0.5 - 1 mm) eingefüllt. MPDA wurde verdampft und mit einem Trägergasstrom von 15 ml/min N2 bei einem Druck von ca. 1 bar und einer Temperatur von 320°C über den Katalysator geleitet. Die MHSV lag zwischen 1 und 2 g MPDA pro Gramm ZSM-5 pro Stunde. Das verwendete MPDA war ein kommerzielles Produkt, das von der Firma Du Pont de Nemours unter dem Handelsnamen Dytek A erhältlich ist. Das Produkt aus dem Cyclisierungs-Reaktor wurde in der Gasphase gehalten und direkt auf einen zweiten Reaktor geleitet. Dieser Reaktor enthielt 12 g eines Dehydrierkatalysators der Zusammensetzung Pd + MgCl₂ auf einem Al₂O₃-Träger (Korngrösse: 0,32 - 1 mm). Die Reaktionsbedingungen waren 280°C und ca. 1 bar. Das Kondensat aus dem Dehydrierreaktor enthielt nach 220 Stunden Reaktionszeit 99,1% 3-Picolin und 0,9% MPI (gaschromatographisch). Eine Desaktivierung der beiden Katalysatoren über die Reaktionszeit war nicht zu beobachten.

### Beispiel 1d

### 2-Methyl-1,5-diaminopentan (MPDA) zu 3-Picolin kontinuierlich in 2 Stufen:

In einen Reaktor (13 mm ⌀) wurden 3 g SiO₂/Al₂O₃-Granulat (Si-HP-87-069 T von Engelhard) in der Korngrösse 0,315 - 1 mm eingefüllt. MPDA wurde verdampft und mit einem Trägergasstrom von 15 ml/min H₂ bei einem Druck von *ca.* 1 bar und einer Reaktortemperatur von 320 °C über den Katalysator geleitet und zu MPI cyclisiert. Das verwendete MPDA war ein kommerzielles Produkt, das von der Firma Du Pont de Nemours unter dem Handelsnamen Dytek A erhältlich ist. Das Produkt aus dem Cyclisierungs-Reaktor wurde in der Gasphase gehalten und direkt auf einen zweiten Reaktor geleitet. Dieser Reaktor enthielt 3 g des Dehydrierkatalysators aus Beispiel 18 der WO 94 / 22 824 (Korngrösse: 0,32-1 mm). Die Reaktortemperatur war 280 °C, der Druck 1 bar. Im Laufe des Versuchs wurde das Edukt von MPDA zu MPI und dann zu einer Rohware (3-MP Roh) gewechselt, die aus einem Gemisch folgender Zusammensetzung besteht: 74,9% MPI, 13,9% MPDA, 5,1% organische Verunreinigungen (hauptsächlich Methylcyclopentandiamine) und 6,1% Wasser. Die Ergebnisse mit den zugehörigen MHSV's (MHSV bezogen auf Reaktor 1) sind in der folgenden Tabelle zusammengestellt:

| **Edukt** | **MHSV [1/h]** | **PIC** | **MPI** | **Laufzeit [h]** | **Desaktivierung [PIC %/h]** |
|---|---|---|---|---|---|
| | | **[GC-Fl.-%]** | | | |
| Dytek A | 2,1 | 99,7 | - | 71 | 0 |
| -"- | 3,15 | 99,6 | 0,2 | 25 | 0 |
| -"- | 4,2 | 98,6 | 1,4 | 48 | 0 |
| MPI | 4,1 | 95,2 | 3,8 | 3 | - |
| -"- | 3,52 | 98,6 | 0,6 | 92 | 0 |
| 3-MP Roh | 4,2 | 93,9 | 1,5 | 170 | 0,0172 |

### Beispiel 2a

### Ammonoxidation von 3-Picolin zu 3-Cyanpyridin:

36,4 g Vanadiumpentoxid, 48,0 g Titandioxid, 197,2 g Zirkoniumdioxid und 0,42 g Molybdäntrioxid wurden in einer Kugelmühle gemahlen. Das Molverhältnis V₂O₅:TiO₂: ZrO₂ entsprach 1:3:8 mit einem Gehalt von 1,15 Gew.% MoO₃ bezogen auf V₂O₅. Aus der Mischung wurden Granalien der Dimension 5 x 5 mm geformt. Diese wurden einer Temperaturbehandlung (100 ° - 120 °C, während 6 h im Luftstrom) unterzogen.
Der so vorbehandelte Katalysator wurde in einer Menge von 60 cm³ (82 g) in einen Rohrreaktor (Edelstahl, Innendurchmesser 20 mm, Länge 1000 mm) eingefüllt.
Bei einer Katalysatorbett-Temperatur von 330 °C wurde darauf eine Mischung von 3-Picolin, Luft und Ammoniak mit einer Zufuhrrate von (g pro l Katalysator pro Stunde = gl⁻¹h⁻¹) 3-Picolin 84 gl⁻¹h⁻¹; Luft 2000 l; Ammoniak 9,92 gl⁻¹h⁻¹ über den Katalysator geleitet. Die molare Zusammensetzung des Zufuhrgases war 3-Picolin:O₂:NH₃ = 1:40:1,3. Entsprechend wurden 25,5 g 3-Picolin über 10 Stunden über den Katalysator geleitet. Der Umsatz war 100%. Es wurden 26,8 g 3-Cyanpyridin entsprechend einer Ausbeute von 95,0% erhalten.

### Beispiel 2b

### Ammonoxidation von 3-Picolin zu 3-Cyanpyridin (In einem Multirohrreaktor ohne zusätzliche thermische Behandlung des Katalysators)

11,67 kg Vanadiumpentoxid, 25,12 kg Titanoxid als Metatitansäure, 63,22 kg Zirkonoxid und 1124 g Molybdäntrioxid (als Ammonparamolybdat) wurden in einer Kugelmühle gemahlen. Das Molverhältnis V₂O₅:TiO₂:ZrO₂ entsprach 1:4:8 mit einem Gehalt von 1,13% MoO₃ bezogen auf V₂O₅. Aus der Mischung wurde ein Granulat mit der Dimension 6 x 6 mm geformt. Diese wurden einer Temperaturbehandlung (100 - 120 °C, während 6 h im Luftstrom) unterzogen.
Eine Quantität (72 kg, 53 Liter) wurde in einem Rohrreaktor (Edelstahl, Innendurchmesser 21 mm, Länge 3000 mm, Anzahl Rohre 51) eingefüllt. Bei einer Katalysatorbett-Temperatur von 340 °C wurde darauf eine Mischung von 3-Picolin, Luft, rückgeführtes Abgas und Ammoniak mit einer Zufuhrrate von (g pro 1 Katalysator pro Stunde = gl⁻¹h⁻¹) 3-Picolin 3,1 kgh-1, (60 gl⁻¹h⁻¹), Luft 7,6 kgh⁻¹, Abgas 67,0 kgh⁻¹, Ammoniak 0,84 kgh-1 über den Katalysator geleitet. Die molare Zusammensetzung des Zufuhrgases war 3-Picolin:O₂:NH₃ 1:1,9:1,5. Entsprechend wurden 1860 kg 3-Picolin über 600 h über den Katalysator geleitet. Es wurden 1880 kg 3-Cyanpyridin entsprechend einer Ausbeute von 90,4% erhalten.

### Beispiel 2c

### Ammonoxidation von 3-Picolin zu 3-Cyanpyridin (In einem Einzelrohrreaktor mit zusätzlicher thermischer Behandlung des Katalysators)

Eine Quantität des von Beispiel 2b erhaltenen Katalysators (135 cm³, 160g) wurde thermisch bei 620°C über 6 h in einem Luftstrom behandelt. Diese wurde anschliessend in einen Rohrreaktor (Innendurchmesser 21 mm, Länge 1000 mm) eingefüllt. Bei einer Katalysatorbett-Temperatur von 375 °C wurde ein Gemisch von 3-Picolin, Luft, Stickstoff und Ammoniak über den Katalysator geleitet. Die Zuführrate war 3-Picolin 11 gh⁻¹, (entpricht 81 g Picolin pro Liter Katalysator pro h), Luft 30 1h⁻¹, Stickstoff 285 1h⁻¹, Ammoniak 4 gh⁻¹, einem Molverhältnis entsprechend von 3-Picolin:O₂:NH₃ 1:2:2,6. Nach 24 h wurden 264 g Picolin über das Katalysatorbett zugeleitet. Der Umsatz betrug 99%. 261 g 3-Cyanpyridin wurden mit einer Ausbeute von 89% erhalten. Die Leistung von 3-Cyanpyridin betrug 80 gl⁻¹h⁻¹.

### Beispiel 2d

### Ammonoxidation von 3-Picolin zu 3-Cyanpyridin (In einem Einzelrohrreaktor mit kleinerem Granulat und höherer Picolin-Leistung)

Aus dem von Beispiel 2b erhaltenen Katalysator wurde ein Granulat mit der Dimension zwischen 3 - 4 mm geformt. Eine Quantität (1 Liter, 1,50 kg) wurde in einen Rohrreaktor (Edelstahl, Innendurchmesser 21 mm, Länge 3000 mm) eingefüllt. Ein Gemisch von 3-Picolin, Luft, Stickstoff und Ammoniak wurde bei einer Katalysatorbett-Temperatur von 353 °C über den Katalysator geleitet. Die Zufuhrrate betrug 3-Picolin 96 gh-1 (entpricht 96 g Picolin pro Liter Katalysator pro h), Luft 210 1h⁻¹, Stickstoff 1340 1h⁻¹, Ammoniak 60 gh⁻¹. Entsprechend wurden 2305 g 3-Picolin über 24 h über das Katalysatorbett geleitet. Es wurden 2380 g 3-Cyanpyridin entsprechend einer Ausbeute von 90% erhalten. Der 3-Picolin-Umsatz betrug 97,5%.

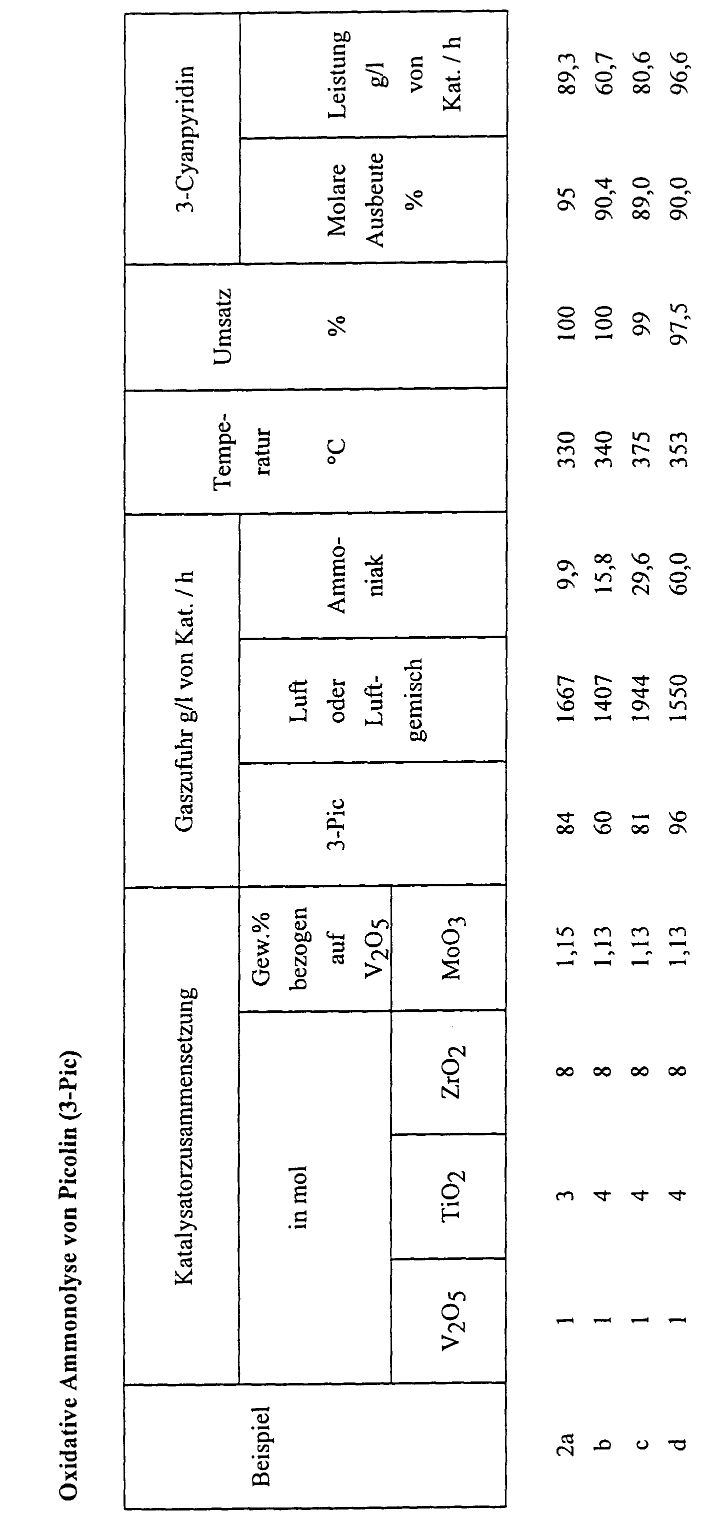

### Beispiel 3a

### Herstellung von NSA aus 3-Cyanpyridin

In einer Kaskade, bestehend aus einem 1,125 1-Reaktor und zwei 0,375 l-Reaktoren, wurde eine Lösung von 10% 3-Cyanpyridin zu NSA umgesetzt. Bei einer Durchflussrate von 300 ml/h Eduktlösung wurde Cyanpyridin quantitativ zu NSA umgesetzt, wobei der erste Reaktor 45 g immobilisierte Mikroorganismen (Trockengewicht) und die beiden weiteren Reaktoren je 7,5 g (Trockengewicht) immobilisierte Mikroorganismen enthielten. Der Biokatalysator blieb während dem ganzen Experiment in den jeweiligen Reaktoren. Der Biokatalysator enthielt immobilisierte Mikroorganismen der Spezies *Rhodococcus rhodochrous* J1.

Die Umsetzung geschah bei einer Temperatur von 25 ± 1 °C und einem pH von 8 bis 8,5. Die Einstellung des pH geschah mit Phosphorsäure und Natronlauge. Die Umsetzung von Cyanpyridin lief in diesem Experiment während 2400 h, ohne dass der Produktstrom mehr als 0,05% Cyanpyridin enthielt, was einem Umsatz von > 99,5% entspricht. Die Aktivität des Katalysators war nach dieser Zeit erschöpft.

Die Produktelösung mit 14 bis 15% NSA wurde über einen 0,2 µm Sterilfilter filtriert. Die erhaltene klare Produketlösung wurde anschliessend bis zur Trockene eingedampft. Der Gehalt an NSA im erhaltenen Produkt war >99,7% (Titration) und entsprach Pharmaqualität.

### Beispiel 3b

### Herstellung von NSA aus 3-Cyanpyridin

In einer Kaskade, bestehend aus einem 150 l-Reaktor und zwei 45 1-Reaktoren, wurde eine Lösung von 15% 3-Cyanpyridin zu NSA umgesetzt. Bei einer Durchflussrate von 25 l/h Eduktlösung wurde Cyanpyridin quantitativ zu NSA umgesetzt, wobei der erste Reaktor 6 kg immobilisierte Mikroorganismen (Trockengewicht) und die beiden weiteren Reaktoren je 0,9 kg (Trockengewicht) immobilisierte Mikroorganismen enthielten. Der Biokatalysator blieb während dem ganzen Experiment in den jeweiligen Reaktoren. Der Biokatalysator enthielt immobilisierte Mikroorganismen der Spezies *Rhodococcus rhodochrous* J1.

Die Umsetzung geschah bei einer Temperatur von 24 ± 2 °C und einem pH von 7 bis 8,5. Die Einstellung des pH geschah mit Phosphorsäure und Natronlauge, wobei auch Kaliumdihydrogenphosphat zur Pufferung verwendet wurde (1 - 3 mg/l).

Die Umsetzung von Cyanpyridin lief in diesem Experiment während 1800 h, ohne dass der Produktstrom mehr als 0,1% Cyanpyridin enthielt, was einem Umsatz von > 99,0% entspricht. Die Aktivität des Katalysators war nach dieser Zeit erschöpft.

Die Produktelösung mit 18 bis 20% NSA wurde anschliessend in Festbettadsorbern (je 15,7 1 Volumen) kontinuierlich gereinigt, wobei zwischen 0,5 bis 4% Aktivkohle (bezogen auf Produktemenge) und 0,5 bis 2% Amberlite XAD2 verwendet wurde.

Die gereinigte NSA-Lösung wurde anschliessend kontinuierlich filtriert. Zum Einsatz gelangte ein dreistufiges Filtrationssystem. Dabei wurde die Produktelösung zuerst einem GAF-Filter (10 - 30 µm Porengrösse), anschliessend einem Sterilfilter (Porengrösse 0,2 µm) und schliesslich einer Ultrafiltration (Porengrösse von 10000 bis 30000 Dalton) zugeführt.

Die filtrierte Produktelösung wurde in einem Fallfilmverdampfer auf 60 bis 80% NSA aufkonzentriert. Vom Produkt entferntes Wasser konnte in der Biohydrolyse rückgeführt werden. Die Produkteisolation wurde in einem Sprühtrockner mit integrierter Wirbelschicht (fluidized spray dryer) durchgeführt.

Der Gehalt an NSA im erhaltenen Produkt war >99,7% (Titration) und entsprach Pharmaqualität.

## Patentansprüche

1. Verfahren zur Herstellung von Nikotinsäureamid,**dadurch gekennzeichnet, dass** in der ersten Stufe
a) 2-Methyl-1,5-diaminopentan in der Gasphase bei 300 ° - 400 °C und bei 0 - 10 bar Überdruck durch Überleiten über einen Katalysator, der als aktive Komponente mindestens ein Oxid von Al und/oder Si enthält, an der Oberfläche ein Verhältnis von sauren zu basischen Zentren von über 2 aufweist und dessen spezifische Oberfläche über
40 m²/g liegt, in das 3-Methylpiperidin überführt wird, welches unmittelbar anschliessend bei 220 ° - 400 °C über einen Dehydrierungskatalysator geleitet und in das 3-Picolin überführt wird, darauf in der zweiten Stufe
b) das 3-Picolin in Gegenwart von Ammoniak und einem Sauerstoff enthaltenden Gas bei 280 ° - 400 °C über einen Ammonoxidationskatalysator geleitet wird, der aus den Oxiden von Vanadium, Titan, Zirkon und Molybdän in einem Molverhältnis V₂O₅ zu TiO₂ zu ZrO₂ von 1:1:2 bis 1:12:25 und einem MoO₃-Gehalt, bezogen auf V₂O₅,von 0,54 Gew.% bis 2,6 Gew.% besteht,und das resultierende 3-Cyanpyridin schliesslich in der dritten Stufe
c) mittels Mikroorganismen der Gattung *Rhodococcus* in das Endprodukt überführt wird.

2. Verfahren nach Patentanspruch 1, **dadurch gekennzeichnet, dass** man als Dehydrierungskatalysator in der ersten Stufe einen Edelmetallkatalysator auf einem Träger verwendet.

3. Verfahren nach Patentansprüchen 1 oder 2, **dadurch gekennzeichnet, dass** als Ammonoxidationskatalysator eine Katalysatorzusammensetzung verwendet wird, die aus den Oxiden von Vanadium, Titan, Zirkon und Molybdän in einem Molverhältnis V₂O₅ zu TiO₂ zu ZrO₂ von 1:3:4 bis 1:8:16 und einem MoO₃-Gehalt, bezogen auf V₂O₅, von 0,54 Gew.% bis 1,20 Gew.% besteht.

4. Verfahren nach mindestens einem der Patentansprüche 1 bis 3, **dadurch gekennzeichnet, dass** in der zweiten Stufe 3-Picolin, Ammoniak und das Sauerstoff enthaltende Gas (berechnet auf O₂) in einem Molverhältnis von 1:1:1,5 bis 1:8,5:60 bei 310 ° - 380 °C über den Katalysator geleitet werden.

5. Verfahren nach Patentanspruch 4, **dadurch gekennzeichnet, dass** 3-Picolin, Ammoniak und das Sauerstoff enthaltende Gas (berechnet auf O₂) in einem Molverhältnis von 1:1:10 bis 1:4:60 bei 310 ° bis 380 °C über den Katalysator geleitet werden.

6. Verfahren nach mindestens einem der Patentansprüche 1 bis 5, **dadurch gekennzeichnet, dass** die mikrobiologische Umsetzung in der dritten Stufe mit Mikroorganismen der Spezies *Rhodococcus rhodochrous* oder mit den funktionell äquivalenten Varianten und Mutanten davon durchgeführt wird.

7. Verfahren nach Patentanspruch 6, **dadurch gekennzeichnet, dass** immobilisierte Mikroorganismen der Spezies *Rhodococcus rhodochrous* verwendet werden.

8. Verfahren nach mindestens einem der Patentansprüche 1 bis 7, **dadurch gekennzeichnet, dass** die mikrobiologische Umsetzung in der dritten Stufe bei einem pH-Wert von 6 bis 10 und einer Temperatur von 5 bis 50 °C durchgeführt wird.

9. Verfahren nach mindestens einem der Patentansprüche 1 bis 8, **dadurch gekennzeichnet, dass** die mikrobiologische Umsetzung in der dritten Stufe in einer Reaktorkaskade, bestehend aus 2 bis 5 miteinander verbundenen gerührten Reaktoren, durchgeführt wird.

10. Verfahren zur Herstellung von Nikotinsäureamid, **dadurch gekennzeichnet, dass** 3-Picolin in Gegenwart von Ammoniak, einem Sauerstoff enthaltenden Gas und bei 280 ° - 400 °C über einen Ammonoxidationskatalysator geleitet wird, der aus den Oxiden von Vanadium, Titan, Zirkon und Molybdän in einem Molverhältnis V₂O₅ zu TiO₂ zu ZrO₂ von 1:1:2 bis 1:12:25 und einem MoO₃-Gehalt, bezogen auf V₂O₅,von 0,54 Gew.% bis 2,6 Gew.% besteht, und das resultierende 3-Cyanpyridin anschliessend mittels Mikroorganismen der Gattung *Rhodococcus* in das Endprodukt überführt wird.

11. Verfahren nach Patentanspruch 10, **dadurch gekennzeichnet, dass** als Ammonoxidationskatalysator eine Katalysatorzusammensetzung verwendet wird, die aus den Oxiden von Vanadium, Titan, Zirkon und Molybdän in einem Molverhältnis V₂O₅ zu TiO₂ zu ZrO₂ von 1:3:4 bis 1:8:16 und einem MoO₃-Gehalt,bezogen auf V₂O₅, von 0,54 Gew.% bis 1,20 Gew.% besteht

12. Verfahren nach Patentanspruch 10 oder 11, **dadurch gekennzeichnet, dass** 3-Picolin, Ammoniak und das Sauerstoff enthaltende Gas (berechnet auf O₂) in einem Molverhältnis von 1:1:1,5 bis 1:8,5:60 bei 310 ° - 380 °C über den Katalysator geleitet werden.

13. Verfahren nach Patentanspruch 12, **dadurch gekennzeichnet, dass** 3-Picolin, Ammoniak und das Sauerstoff enthaltende Gas (berechnet auf O₂) in einem Molverhältnis von 1:1:10 bis 1:4:60 über den Katalysator geleitet werden.

14. Verfahren nach mindestens einem der Patentansprüche 10 bis 13, **dadurch gekennzeichnet, dass** die mikrobiologische Umsetzung mit Mikroorganismen der Spezies *Rhodococcus rhodochrous* oder mit den funktionell äquivalenten Varianten und Mutanten davon durchgeführt wird.

15. Verfahren nach Patentanspruch 14, **dadurch gekennzeichnet, dass** immobilisierte Mikroorganismen der Spezies *Rhodococcus rhodochrous* verwendet werden.

16. Verfahren nach mindestens einem der Patentansprüche 10 bis 15, **dadurch gekennzeichnet, dass** die mikrobiologische Umsetzung bei einem pH-Wert von 6 bis 10 und einer Temperatur von 5 bis 50 °C durchgeführt wird.

17. Verfahren nach mindestens einem der Patentansprüche 10 bis 16, **dadurch gekennzeichnet, dass** die mikrobiologische Umsetzung in einer Reaktorkaskade, bestehend aus 2 bis 5 miteinander verbundenen gerührten Reaktoren, durchgeführt wird.

## Claims

1. Process for preparing nicotinamide, **characterized in that** in the first stage
a) 2-methyl-1,5-diaminopentane in the gas phase at 300°-400°C and at 0-10 bar gauge pressure is converted into 3-methylpiperidine by passing it over a catalyst containing as active component at least one oxide of Al and/or Si, having at the surface a ratio of acid centres to basic centres of more than 2 and having a specific surface area of more than 40 m²/g, and immediately afterwards the 3-methylpiperidine is passed at 220°-400°C over a dehydrogenation catalyst and is converted into 3-picoline, then in the second stage
b) the 3-picoline is, in the presence of ammonia and an oxygen-containing gas, passed at 280°-400°C over an ammonoxidation catalyst comprising the oxides of vanadium, titanium, zirconium and molybdenum in a molar ratio of V₂O₅ to TiO₂ to ZrO₂ of from 1:1:2 to 1:12:25 and having an MoO₃ content, based on V₂O₅, of from 0.54% by weight to 2.6% by weight, and finally in the third stage
c) the resulting 3-cyanopyridine is converted by means of microorganisms of the genus Rhodococcus into the end product.

2. Process according to Claim 1, **characterized in that** the dehydrogenation catalyst used in the first stage is a noble metal catalyst on a support.

3. Process according to Claim 1 or 2, **characterized in that** the ammonoxidation catalyst used is a catalyst composition comprising the oxides of vanadium, titanium, zirconium and molybdenum in a molar ratio of V₂O₅ to TiO₂ to ZrO₂ of from 1:3:4 to 1:8:16 and having an MoO₃ content, based on V₂O₅, of from 0.54% by weight to 1.20% by weight.

4. Process according to at least one of Claims 1 to 3, **characterized in that** in the second stage 3-picoline, ammonia and the oxygen-containing gas (calculated as O₂) are passed in a molar ratio of from 1:1:1.5 to 1:8.5:60 at 310° to 380°C over the catalyst.

5. Process according to Claim 4, **characterized in that** 3-picoline, ammonia and the oxygen-containing gas (calculated as O₂) are passed in a molar ratio of from 1:1:10 to 1:4:60 at from 310° to 380°C over the catalyst.

6. Process according to at least one of Claims 1 to 5, **characterized in that** the microbiological conversion in the third stage is carried out using microorganisms of the species *Rhodococcus rhodochrous* or using the functionally equivalent variants and mutants thereof.

7. Process according to Claim 6, **characterized in that** immobilized microorganisms of the species *Rhodococcus rhodochrous* are used.

8. Process according to at least one of Claims 1 to 7, **characterized in that** the microbiological conversion in the third stage is carried out at a pH of from 6 to 10 and a temperature of from 5 to 50°C.

9. Process according to at least one of Claims 1 to 8, **characterized in that** the microbiological conversion in the third stage is carried out in a reactor cascade consisting of from 2 to 5 connected stirred reactors.

10. Process for preparing nicotinamide, **characterized in that** 3-picoline is, in the presence of ammonia and an oxygen-containing gas, passed at 280°-400°C over an ammonoxidation catalyst comprising the oxides of vanadium, titanium, zirconium and molybdenum in a molar ratio of V₂O₅ to TiO₂ to ZrO₂ of from 1:1:2 to 1:12:25 and having an MoO₃ content, based on V₂O₅, of from 0.54% by weight to 2.6% by weight and the resulting 3-cyanopyridine is subsequently converted by means of microorganisms of the genus *Rhodococcus* into the end product.

11. Process according to Claim 10, **characterized in that** the ammonoxidation catalyst used is a catalyst composition comprising the oxides of vanadium, titanium, zirconium, and molybdenum in a molar ratio of V₂O₅ to TiO₂ to ZrO₂ of from 1:3:4 to 1:8:16 and having an MoO₃ content, based on V₂O₅, of from 0.54% by weight to 1.20% by weight.

12. Process according to Claim 10 or 11, **characterized in that** 3-picoline, ammonia and the oxygen-containing gas (calculated as O₂) are passed in a molar ratio of from 1:1:1.5 to 1:8.5:60 at 310° to 380°C over the catalyst.

13. Process according to Claim 12, **characterized in that** 3-picoline, ammonia and the oxygen-containing gas (calculated as O₂) are passed in a molar ratio of from 1:1:10 to 1:4:60 over the catalyst.

14. Process according to at least one of Claims 10 to 13, **characterized in that** the microbiological conversion is carried out using microorganisms of the species *Rhodococcus rhodochrous* or using the functionally equivalent variants and mutants thereof.

15. Process according to Claim 14, **characterized in that** immobilized microorganisms of the species *Rhodococcus rhodochrous* are used.

16. Process according to at least one of Claims 10 to 15, **characterized in that** the microbiological reaction is carried out at a pH of from 6 to 10 and a temperature of from 5 to 50°C.

17. Process according to at least one of Claims 10 to 16, **characterized in that** the microbiological reaction is carried out in a reactor cascade consisting of from 2 to 5 connected stirred reactors.

## Revendications

1. Procédé de préparation d'amide d'acide nicotinique **caractérisé en ce que**, dans la première étape
a) par passage sur un catalyseur qui contient comme composant actif au moins un oxyde de Al et/ou de Si, qui présente à la surface un rapport des centres acides aux centres basiques supérieur à 2 et dont la surface spécifique est supérieure à 40 m²/g, le 2-méthyl-1,5-diaminopentane en phase gazeuse à 300° - 400°C et à une surpression de 0 - 10 bar est converti en la 3-méthylpipéridine qui est amenée à passer immédiatement après à 220° - 400°C sur un catalyseur de déshydrogénation et est convertie en 3-picoline, puis dans la deuxième étape
b) la 3-picoline est amenée à passer en présence d'ammoniac et d'un gaz contenant de l'oxygène à 280° - 400°C sur un catalyseur d'ammoxydation qui consiste en les oxydes de vanadium, de titane, de zirconium et de molybdène dans un rapport molaire de V₂O₅ à TiO₂ à ZrO₂ de 1:1:2 à 1:12:25 et une teneur en MoO₃, par rapport à V₂O₅, de 0,54 % en masse à 2,6 % en masse, et enfin, dans la troisième étape, la 3-cyanopyridine résultante est
c) convertie en le produit final au moyen de micro-organismes du genre *Rhodococcus.*

2. Procédé selon la revendication 1 **caractérisé en ce que** l'on utilise comme catalyseur de déshydrogénation dans la première étape un catalyseur à base de métal noble sur un support.

3. Procédé selon les revendications 1 ou 2 **caractérisé en ce que** l'on utilise comme catalyseur d'ammoxydation une composition catalytique qui consiste en les oxydes de vanadium, de titane, de zirconium et de molybdène dans un rapport molaire de V₂O₅ à TiO₂ à ZrO₂ de 1:3:4 à 1:8:16 et une teneur en MoO₃, par rapport à V₂O₅, de 0,54 % en masse à 1,20 % en masse.

4. Procédé selon au moins l'une des revendications 1 à 3 **caractérisé en ce que**, dans la deuxième étape, on fait passer sur le catalyseur la 3-picoline, l'ammoniac et le gaz contenant de l'oxygène (calculé en O₂) dans un rapport molaire de 1:1:1,5 à 1:8,5:60 à 310° - 380°C.

5. Procédé selon la revendication 4 **caractérisé en ce que** l'on fait passer sur le catalyseur la 3-picoline, l'ammoniac et le gaz contenant de l'oxygène (calculé en O₂) dans un rapport molaire de 1:1:10 à 1:4:60 à 310° à 380°C.

6. Procédé selon au moins l'une des revendications 1 à 5 **caractérisé en ce que** la conversion microbiologique dans la troisième étape est réalisée avec des micro-organismes de l'espèce *Rhodococcus rhodochrous* ou avec leurs variants et mutants équivalents du point de vue fonctionnel.

7. Procédé selon la revendication 6 **caractérisé en ce que** l'on utilise des micro-organismes immobilisés de l'espèce *Rhodococcus rhodochrous.*

8. Procédé selon au moins l'une des revendications 1 à 7 **caractérisé en ce que** la conversion microbiologique dans la troisième étape est réalisée à un pH de 6 à 10 et à une température de 5 à 50°C.

9. Procédé selon au moins l'une des revendications 1 à 8 **caractérisé en ce que** la conversion microbiologique dans la troisième étape est réalisée dans une cascade de réacteurs consistant en 2 à 5 réacteurs agités reliés entre eux.

10. Procédé de préparation d'amide d'acide nicotinique **caractérisé en ce que** la 3-picoline est amenée à passer en présence d'ammoniac, d'un gaz contenant de l'oxygène et à 280° - 400°C sur un catalyseur d'ammoxydation qui consiste en les oxydes de vanadium, de titane, de zirconium et de molybdène dans un rapport molaire de V₂O₅ à TiO₂ à ZrO₂ de 1:1:2 à 1:12:25 et une teneur en MoO₃, par rapport à V₂O₅, de 0,54 % en masse à 2,6 % en masse, et la 3-cyanopyridine résultante est ensuite convertie en le produit final au moyen de micro-organismes du genre *Rhodococcus.*

11. Procédé selon la revendication 10 **caractérisé en ce que** l'on utilise comme catalyseur d'ammoxydation une composition catalytique qui consiste en les oxydes de vanadium, de titane, de zirconium et de molybdène dans un rapport molaire de V₂O₅ à TiO₂ à ZrO₂ de 1:3:4 à 1:8:16 et une teneur en MoO₃, par rapport à V₂O₅, de 0,54 % en masse à 1,20 % en masse.

12. Procédé selon la revendication 10 ou 11 **caractérisé en ce que** l'on fait passer sur le catalyseur la 3-picoline, l'ammoniac et le gaz contenant de l'oxygène (calculé en O₂) dans un rapport molaire de 1:1:1,5 à 1:8,5:60 à 310° - 380°C.

13. Procédé selon la revendication 12 **caractérisé en ce que** l'on fait passer sur le catalyseur la 3-picoline, l'ammoniac et le gaz contenant de l'oxygène (calculé en O₂) dans un rapport molaire de 1:1:10 à 1:4:60.

14. Procédé selon au moins l'une des revendications 10 à 13 **caractérisé en ce que** la conversion microbiologique est réalisée avec des micro-organismes de l'espèce *Rhodococcus rhodochrous* ou avec leurs variants et mutants équivalents du point de vue fonctionnel.

15. Procédé selon la revendication 14 **caractérisé en ce que** l'on utilise des micro-organismes immobilisés de l'espèce *Rhodococcus rhodochrous.*

16. Procédé selon au moins l'une des revendications 10 à 15 **caractérisé en ce que** la conversion microbiologique est réalisée à un pH de 6 à 10 et à une température de 5 à 50°C.

17. Procédé selon au moins l'une des revendications 10 à 16 **caractérisé en ce que** la conversion microbiologique est réalisée dans une cascade de réacteurs consistant en 2 à 5 réacteurs agités reliés entre eux.
